# EUROPEAN PATENT SPECIFICATION

(11) **EP 3 352 723 B1**
(45) Date of publication and mention of the grant of the patent: **06.03.2019**
(21) Application number: 16762992.2
(22) Date of filing: 30.08.2016
(51) Int. Cl.: A61K 6/033, A61K 8/29, A61Q 11/00, A61K 8/19

(54) **ORAL CARE COMPOSITION**
MUNDPFLEGEZUSAMMENSETZUNG
COMPOSITION DE PROTECTION ORALE

(30) Priority: 24.09.2015 WO PCT/CN2015/090545; 27.10.2015 EP 15191562
(43) Date of publication of application: 01.08.2018
(73) Proprietor: Unilever NV, 3013 AL Rotterdam (NL); Unilever PLC, London Greater London EC4Y 0DY (GB)
(72) Inventor: LI, Xiaoke, Shanghai 200335 (CN); QUAN, Yan, Shanghai 200335 (CN)
(74) Representative: Tansley, Sally Elizabeth
(86) International application number: PCT/EP2016/070347
(87) International publication number: WO 2017/050528

(56) References cited:
- EP-A1- 2 340 806
- WO-A1-2014/074808
- WO-A2-2012/031785

## Description

### Field of the Invention

The present invention relates to oral care compositions such as tooth pastes, gums, mouthwashes and the like. In particular the present invention relates to oral care compositions containing a particulate tooth whitening agent. The invention also relates to the use of such compositions for whitening and /or remineralizing of teeth of an individual.

### Background of the Invention

The enamel layer of the tooth is naturally an opaque white or slightly off-white colour. However, this enamel layer can become stained or discoloured.

Many products we consume have a negative impact on our teeth and mouth. Many substances can stain or reduce the whiteness of one's teeth, in particular, certain foods, tobacco products, and fluids such as tea and coffee. These staining and discolouring substances are often able to permeate the enamel layer. This problem occurs gradually over many years, but imparts a noticeable discoloration of the enamel of one's teeth.

Consumers have always had a strong desire for white teeth and many individuals are dissatisfied with their current teeth colour. This desire for whiter teeth has given rise to a growing trend in the increased use of tooth whitening products.

A variety of products are currently used for teeth whitening. Such products often comprise peroxides, abrasives or both in order to clean and whiten teeth. These types of products are often not desired since they do not contribute to the remineralization of teeth and can cause damage to teeth and gums if overused. Products comprising calcium source have been developed in an attempt to enhance the characteristics of teeth. Such products, however, do not positively adhere to teeth and thereby may only contact teeth for a brief period of time prior to being rinsed out of the mouth in wash water.

The present inventors have now recognized a need to develop an oral care composition which has particulate tooth whitening agents that are suitable to adhere to the tooth surfaces to provide tooth whitening benefits. Particularly, the particulate tooth whitening agents are composite particles that are found to effectively deposit on tooth surfaces even during routine oral hygiene practices such as brushing, especially the composite particles having a second component coating comprising a mixture of 1) metal oxides and/or hydrated metal oxides and 2) solid non-metal oxides and/or solid hydrated non-metal oxides. The present inventors have found unexpectedly that oral care composition comprising such composite particles showed excellent tooth whitening benefit in the presence of calcium silicate, which behaves as a deposition aid to enhance the deposition of such composite particles on tooth surfaces.

### Additional Information

WO 2008/068149 A (Unilever) discloses an oral care product comprising a first composition comprising an insoluble calcium salt that is not a calcium phosphate salt, a second independent composition comprising a source of phosphate ions, and a means for delivering each of the compositions to the surface of the teeth. The preferred insoluble calcium salt is calcium silicate.

WO 2012/031785 A (Unilever) discloses composite particle actives suitable for use in oral care compositions. The composite particle actives have a core and a coating whereby the coating interacts with phosphate ions to produce calcium and phosphate reaction products that are suitable to adhere to tooth enamel and/or dentin in order to improve characteristics of teeth.

WO 2012/031786 A (Unilever) discloses oral care compositions with composite particle actives described in WO 2012/031785 A (Unilever).

None of the references above describes an oral care composition comprising calcium silicate and composite particles, wherein the composite particles have a first component core comprising a metal compound having a refractive index in the range from 1.9 to 4.0, and a second component coating comprising a mixture of 1) metal oxides and/or hydrated metal oxides and 2) solid non-metal oxides and/or solid hydrated non-metal oxides.

### Test and definitions

### Dentifrice

"Dentifrice" for the purposes of the present invention means a paste, powder, liquid, gum or other preparation for cleaning the teeth or other surfaces in the oral cavity.

### Tooth Paste

"Tooth paste" for the purpose of the present invention means a paste or gel dentifrice for use with a toothbrush. Especially preferred are tooth pastes suitable for cleaning teeth by brushing for about two minutes.

### Mouth Wash

"Mouth wash" for the purpose of the present invention means liquid dentifrice for use in rinsing the mouth. Especially preferred are mouth washes suitable for rinsing the mouth by swishing and/or gargling for about half a minute before expectorating.

### Particle Size

"Particle size" for the purpose of the present invention means D50 particle size. The D50 particle size of a particulate material is the particle size diameter at which 50 wt% of the particles are larger in diameter and 50 wt% are smaller in diameter. For the purpose of the present invention, particle sizes and distribution are measured using Malvern Mastersizer 2000 and Malvern ZetaSizer Nano series.

### Composite Particle

"Composite particle" for the purpose of the present invention means a particle comprising a first component core and a second component coating wherein the core and coating are composed of different materials.

### Hydrated Oxides

"Hydrated oxides" for the purpose of the present invention means a compound of an oxide with water, wherein the water is chemically combined with the oxide in a way that it can be removed by heating. "Oxide" for the purpose of the present invention means a chemical compound that contains at least one oxygen atom and one other element in its chemical formula.

### Deposition Aid

"Deposition aid" for the purpose of the present invention means a material which aids deposition of particulate tooth whitening agent from the continuous phase of the composition onto the tooth surfaces during use of the composition.

### Refractive Index

Refractive index is quoted at a temperature of 25°C and a wavelength of 589 nm.

### pH

pH is quoted at atmospheric pressure and a temperature of 25°C. When referring to the pH of an oral care composition, this means the pH measured when 5 parts by weight of the composition is uniformly dispersed and/or dissolved in 20 parts by weight pure water at 25°C. In particular the pH may be measured by manually mixing 5 g oral care composition with 20 mL water for 30 s, then immediately testing the pH with indicator or a pH meter.

### Solubility

"Soluble" and "insoluble", as used herein, refers to the solubility of a source (e.g., like calcium salts) in water at 25°C and atmospheric pressure. "Soluble" means a source that dissolves in water to give a solution with a concentration of at least 0.1 moles per litre. "Insoluble" means a source that dissolves in water to give a solution with a concentration of less than 0.001 moles per litre. "Sparingly soluble", therefore, is defined to mean a source that dissolves in water to give a solution with a concentration of greater than 0.001 moles per litre and less than 0.1 moles per litre.

### Water of Hydration

"Water of hydration" for the purpose of the present invention means water chemically combined with a substance in a way that it can be removed by heating without substantially changing the chemical composition of the substance. In particular, water which could only be removed when heated above 200°C. The water loss is measured using thermo gravimetric analysis (TGA) with a Netzsch TG instrument. The TGA is conducted under an N₂ atmosphere with heating rate of 10 degree/min in the range of 30 to 900°C.

### Substantially Free

"Substantially free of' for the purpose of the present invention means less than 1.5%, and preferably less than 1.0%, and more preferably less than 0.75% and more preferably still less than 0.5%, and even more preferably less than 0.1% and most preferably from 0.0 to 0.01% by weight, based on total weight of the oral care composition, including all ranges subsumed therein.

### Dual-Phase

"Dual-Phase" for the purpose of the present invention means a composition having two independent phases which are physically separate.

### Viscosity

Viscosity of a tooth paste is the value taken at room temperature (25°C) with a Brookfield Viscometer, Spindle No.4 and at a speed of 5 rpm. Values are quoted in centipoises (cP=mPa.s) unless otherwise specified.

### Remineralization

"Remineralization" for the purpose of the present invention means *in situ* (i.e. in the oral cavity) generation of calcium phosphate on teeth (including layers on teeth from 10 nm to 20 microns, and preferably from 75 nm to 10 microns, and most preferably, from 150 nm to 5 microns thick including all ranges subsumed therein) to reduce the likelihood of tooth sensitivity, tooth decay, regenerate enamel and/or improve the appearance of teeth by whitening through the generation of such new calcium phosphate.

### Miscellaneous

Except in the examples, or where otherwise explicitly indicated, all numbers in this description indicating amounts of material or conditions of reaction, physical properties of materials and/or use may optionally be understood as modified by the word "about".
All amounts are by weight of the final oral care composition, unless otherwise specified.
It should be noted that in specifying any ranges of values, any particular upper value can be associated with any particular lower value.

For the avoidance of doubt, the word "comprising" is intended to mean "including" but not necessarily "consisting of' or "composed of'. In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### Summary of the Invention

In a first aspect, the present invention is directed to an oral care composition comprising:
a) calcium silicate;
b) composite particles; and
c) a physiologically acceptable carrier;
   wherein the composite particle comprises a first component core comprising a metal compound having a refractive index in the range from 1.9 to 4.0, and a second component coating comprising a mixture of:
   1) metal oxides and/or hydrated metal oxides; and
   2) solid non-metal oxides and/or solid hydrated non-metal oxides.

In a second aspect, the present invention is directed to a packaged oral care product comprising the oral care composition of the first aspect of this invention.

In a third aspect, the present invention is directed to a method of whitening and/or remineralizing of teeth of an individual comprising the step of applying the oral care composition of any embodiment of the first aspect to at least one surface of the teeth of the individual.

All other aspects of the present invention will more readily become apparent upon considering the detailed description and examples which follow.

### Detailed Description of the Invention

### CALCIUM SILICATE

In a preferred embodiment, the calcium silicate used is CaSiO₃ which has low water solubility and is made commercially available under the name Sorbosil CA40 by PQ Corporation. In another preferred embodiment, the calcium silicate is insoluble, present as the composite material calcium oxide-silica (CaO-SiO₂), which is described, for example, in international patent application published as WO 2008/01517(Unilever) which is hereby incorporated by reference in its entirety. For a calcium silicate composite material, the atom ratio of calcium to silicon (Ca:Si) may be from 1:30 to 3:1. The Ca:Si ratio is preferably 1:20 to 2:1, and more preferably, from 1:10 to 1:1, and most preferably, from about 1:7 to 1:1.5. The calcium silicate may comprise mono-calcium silicate, bi-calcium silicate, or tri-calcium silicate. The calcium silicate may be in a crystalline or amorphous state or even in a mesoporous state.

In addition to calcium oxide, silica, the particles comprising the calcium silicate which is not hydrated may comprise other components, such as metal cations, anions (such as phosphate) and the like. However, it is preferred that the particles comprise calcium oxide, silica in an amount of at least 70% by weight of the particles, more preferably at least 80%, more preferably still at least 90% and even more preferably at least 95%. Most preferably the particles consist of (or at least consist essentially of) calcium oxide, silica.

In another preferred embodiment, the calcium silicate is calcium silicate hydrate. The calcium silicate hydrate for use in the present invention comprises at least calcium oxide (CaO), silica (SiO₂) and water. Compared with conventional calcium silicate which are not hydrated, the calcium silicate hydrate comprises the water of hydration in an amount of at least 5% by weight of the calcium silicate hydrate, preferably at least 10%, more preferably at least 15%, even more preferably at least 20% and most preferably at least 25%. The water content is typically no greater than 50% by weight of the calcium silicate hydrate, more preferably no greater than 40%, even more preferably no greater than 35% and most preferably no greater than 30%.

The calcium silicate hydrate preferably comprises at least 20% silica by weight of the calcium silicate hydrate, more preferably at least 30%, more preferably still at least 40% and most preferably at least 55%. The silica content is preferably no greater than 70% by weight of the calcium silicate hydrate, more preferably no greater than 65% and most preferably no greater than 60%.

To provide calcium necessary for remineralization, the calcium silicate hydrate preferably comprises calcium oxide in an amount of at least 5% by weight of the calcium silicate hydrate, more preferably at least 7%, more preferably still at least 10%, even more preferably at least 12% and most preferably at least 15%. The calcium oxide content is typically no greater than 50% by weight of the calcium silicate hydrate, more preferably no greater than 40%, even more preferably no greater than 30% and most preferably no greater than 25%.

The calcium silicate hydrate preferably comprises Ca and Si in an atom ratio (Ca:Si) less than 1:1, more preferably less than 1:1.2, more preferably still from 1:1.5 to 1:4 and most preferably from 1:1.7 to 1:3.

The calcium silicate may be amorphous or at least partly crystalline or mesoporous. The calcium silicate is preferably particulate as this allows for maximum surface area for contact with dental tissue. Thus preferably the composition comprises particles comprising the calcium silicate. Preferably from 10 to 100%, and especially, from 25 to 100%, and most especially, from 70% to 100% by weight of the particles comprising calcium silicate used in this invention have a particle size from 100 nm to less than 50 microns, preferably from 500 nm to 30 microns, more preferably from 1 micron to 20 microns, most preferably from 3 micron to 15 microns.

In addition to calcium oxide, silica and water, the particles which comprise the calcium silicate hydrate may comprise other components, such as metal cations, anions (such as phosphate) and the like. However, it is preferred that the particles comprise CaO, SiO₂ and water in an amount of at least 70% by weight of the particles, more preferably at least 80%, more preferably still at least 90% and even more preferably at least 95%. Most preferably the particles consist of (or at least consist essentially of) CaO, SiO₂ and water.

Typically, the oral care composition of the present invention comprises from 0.1 to 50% by weight of the calcium silicate, more preferably from 0.2 to 30%, most preferably from 1 to 20%, based on the total weight of the oral care composition and including all ranges subsumed therein.

### COMPOSITE PARTICLES

Typically, the particulate tooth whitening agents being used in the invention are composite particles. The only limitation with respect to the composite particle that may be used in this invention is that the same is suitable for use in the mouth.

The composite particle comprises a first component core and a second component coating. Typically, the core of the composite particle comprises a material suitable to whiten teeth. Such a material is preferred to have a refractive index of at least 1.9, more preferably at least 2.0, even more preferably at least 2.2, even more preferably still at least 2.4 and most preferably at least 2.5.

The maximum refractive index of the material is not particularly limited but preferably up to 4.0. It is known that the refractive index of a composite particle comprising more than one material can be calculated based on the refractive indices and volume fractions of the constituents using effective medium theory, as is described for example in WO 2009/023353.

Particular suitable core materials are metal salts and preferred are salts where the metal is selected from zinc (Zn), titanium (Ti), zirconium (Zr) or a combination thereof. Preferably, the metal salts is (or at least comprises) a metal oxide such as titanium dioxide (TiO₂), zinc oxide (ZnO), zirconium dioxide (ZrO₂) or a combination thereof. In addition, the core of the composite particle can also comprise non-metal oxides such as silicon monoxide (SiO).

In a preferred embodiment, the core comprises metal oxides, non-metal oxides or a combination thereof in an amount of at least 50% by weight of the core and more preferably at least 70%, and most preferably from 80 to 100%. In an especially preferred embodiment, the core is at least 50% by weight titanium dioxide, and most preferably, from 60 to 100% by weight titanium dioxide, based on total weight of the first component core.

In an especially preferred embodiment, the core of the composite particle is titanium dioxide. The titanium dioxide can have a crystal structure of anatase or rutile. Rutile is preferred due to its high refractive index. Thus in a preferred embodiment, the titanium dioxide comprises or is rutile.

Typically, the composite particle comprises the core in an amount of at least 50%, more preferably at least 70%, more preferably still from 80 to 99%, most preferably from 85% to 98% by weight of the composite particle, based on total weight of the composite particle and including all ranges subsumed therein.

The second component coating comprises material suitable to adhere to tooth enamel, dentin or both. "Adhere", as used herein, includes bonding and/or effectively coupling to tooth surfaces as a result of the interaction between the elements in the coating and those in teeth.

Typically, the coating material comprises oxides and/or hydrated oxides. Illustrative yet non-limiting examples of the type of the coating material suitable for use in this invention include, for example, alumina, hydrated alumina (aluminium hydroxide), silica, hydrated silica, copper oxide, lanthanum oxide, nickel oxide, lead oxide, zinc oxide, magnesium oxide, mixtures thereof or the like. In a preferred embodiment, the coating material comprises or is a mixture of 1) metal oxides and/or hydrated metal oxides and 2) solid non-metal oxides and/or solid hydrated non-metal oxides, preferably the metal oxide is alumina and the non-metal oxide is silica.

In another preferred embodiment, the coating material comprises or is a mixture of:
1) alumina and/or hydrated alumina; and
2) silica and/or hydrated silica.

In an especially preferred embodiment, the coating material comprises or is a mixture of:
1) alumina and/or hydrated alumina; and
2) silica.

In another especially preferred embodiment, the coating material comprises or is a mixture of:
1) alumina; and
2) silica and/or hydrated silica.

Some specific examples of such composite particles suitable for use in this invention include titanium dioxide and/or zinc oxide coated with a mixture of 1) alumina and/or hydrated alumina and 2) silica and/or hydrated silica.

Titanium dioxide coated with a mixture of hydrated alumina and silica are made commercially available, for example, from suppliers like KOBO Products Inc under the trade name SIH-5 TiO2 R250. Titanium dioxide coated with a mixture of alumina and hydrated silica are made commercially available, for example, from Tayca Corporation under the trade name JR-800.

The tooth enamel surfaces carry some negative charges. The present inventors believe that the particles may carry some positive charge when in contact with oral fluid due to the presence of metal oxides and/or hydrated metal oxides in the second component coating, which improves the interaction with the tooth enamel surface and enhances the deposition onto the tooth enamel surface. Besides, the solid non-metal oxides and/or solid hydrated non-metal oxides in the coating material may have an affinity for Ca ions in teeth, which further enhances the deposition of the particles onto the tooth enamel surface.

Typically, the coating material comprises at least 60% by weight of oxides and/or hydrated oxides, more preferably at least 70%, and most preferably from 80 to 100% by weight of oxides and/or hydrated oxides based on total weight of the second component coating and including all ranges subsumed therein. In a preferred embodiment, the coating material comprises 1) metal oxides and/or hydrated metal oxides and 2) solid non-metal oxides and/or solid hydrated non-metal oxides in a weight ratio ranging from 1:10 to 10:1, preferably from 1:5 to 5:1.

The first component core will typically form the majority of the composite particle and so preferably the particles comprise the first component core and the second component coating in a weight ratio (first component core: the second component coating) of at least 1:1, more preferably at least 2:1, more preferably still at least 3:1 and most preferably ranging from 5:1 to 30:1.

Typically, the composite particles have a size from 10 nm to less than 10 microns, and more preferably, from 50 nm to 1 micron, more preferably still from 100 nm to 500 nm, most preferably from 150 nm to 350 nm.

Typically, the oral care composition of the present invention comprises from 0.25 to 60%, and more preferably, from 0.5 to 40%, and most preferably, from 1 to 30% by weight of the composite particles, based on the total weight of the oral care composition and including all ranges subsumed therein.

Typically, the weight ratio of calcium silicate to composite particles ranges from 1:10 to 4:1, more preferably from 1:5 to 3:1, most preferably from 1:3 to 2:1.

### OPTIONAL COMPONENTS

The composite particles of the present invention may be capable of adhering to tooth surfaces in the presence of calcium silicate even without inclusion of a phosphate source in the oral care composition itself. Without wishing to be bound by theory the present inventors believe that this may be because the calcium silicate in the oral composition of the present invention reacts with phosphate ions in saliva and/or Si-OH groups may have an affinity for Ca ions in teeth. The remineralization of calcium silicate around the composite particles helps the deposition of composite particles on tooth surface by enhancing their resistance to shear force.

Thus in one embodiment the oral care composition may be substantially free of phosphate source. This is especially preferred when the composition is a monophase hydrous composition (i.e. comprises greater than 1.5% water, preferably greater than 5% water, more preferably greater than 10% water and most preferably from 20 to 90% water by weight of the composition). Presence of calcium silicate, composite particles and phosphate sources in a monophase hydrous formulation can lead to premature reaction of the calcium and phosphate and instability of the product.

For certain compositions, especially anhydrous compositions (i.e. compositions substantially free of water) or dual-phase hydrous compositions, it is preferable to compound a phosphate source in the oral composition to aid *in situ* generation of calcium phosphate.

The phosphate source that may be used in this invention is limited only to the extent that the same may be used in a composition suitable for use in the mouth. Illustrative examples of the types of phosphate source suitable for use in this invention include trisodium phosphate, monosodium dihydrogen phosphate, disodium hydrogen phosphate, sodium pyrophosphate, tetrasodium pyrophosphate, sodium hexametaphosphate, tripotassium phosphate, monopotassium dihydrogen phosphate, dipotassium hydrogen phosphate, mixtures thereof or the like. The phosphate source is preferably one which is water soluble.

When used, the phosphate source typically makes up from 0.5 to 22%, and preferably, from 2 to 18%, and most preferably, from 4 to 16% by weight of the oral care composition, based on total weight of the oral care composition and including all ranges subsumed therein. In a preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein. In another preferred embodiment, the phosphate source used is or at least comprises monosodium dihydrogen phosphate.

The oral care composition preferably has a pH of greater than 5.0. If the pH of the composition is too low then it may lower the pH in the oral cavity such that generation of *in situ* calcium phosphate is retarded. Therefore, it is preferred that the pH of the oral care composition is in the range 5.5 to 11.0, more preferably 6.0 to 10.5 and most preferably 7.0 to 10.0.

The composition of the present invention is an oral care composition and typically comprises physiologically acceptable carrier. The carrier preferably comprises at least surfactant, thickener, humectant or a combination thereof.

Preferably the oral care composition comprises a surfactant. Preferably the composition comprises at least 0.01% surfactant by weight of the composition, more preferably at least 0.1% and most preferably from 0.5 to 7%. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulphate and/or sodium dodecylbenzene sulfonate. Most preferably the surfactant is sodium lauryl sulphate.

Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, xanthan gum and/or sodium carboxymethyl cellulose and/or a Carbomer is/are preferred. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

In another especially preferred embodiment, the thickener is xanthan gum.

Thickener typically makes up from 0.01 to about 10%, more preferably from 0.1 to 9%, and most preferably, from 1.5 to 8% by weight of the oral care composition, based on total weight of the composition and including all ranges subsumed therein.

When the oral care composition of this invention is a toothpaste or gel, the same typically has a viscosity from about 30,000 to 180,000 centipoise, and preferably, from 60,000 to 170,000 centipoise, and most preferably, from 65,000 to 165,000 centipoise.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the oral care composition. More preferably, the carrier humectant makes up from 25 to 80%, and most preferably, from 45 to 70% by weight of the composition, based on total weight of the composition and including all ranges subsumed therein.

The oral care composition of the present invention may contain a variety of other ingredients which are common in the art to enhance physical properties and performance. These ingredients include antimicrobial agents, anti-inflammatory agents, anti-caries agents, plaque buffers, fluoride sources, vitamins, plant extracts, desensitizing agents, anti-calculus agents, biomolecules, flavors, proteinaceous materials, preservatives, opacifying agents, coloring agents, pH-adjusting agents, sweetening agents, particulate abrasive materials, polymeric compounds, buffers and salts to buffer the pH and ionic strength of the compositions, and mixtures thereof. Such ingredients typically and collectively make-up less than 20% by weight of the composition, and preferably, from 0.0 to 15% by weight, and most preferably, from 0.01 to 12% by weight of the composition, including all ranges subsumed therein.

The oral care composition of this invention can be used in a method of whitening and/or remineralizing of teeth of an individual comprising applying the composition to at least one surface of the teeth of the individual. The oral care composition of this invention may additionally or alternatively be for use as a medicament and/or used in the manufacture of a medicament for providing an oral care benefit as described herein, such as for whitening the teeth of an individual. Alternatively and preferably, the use is non-therapeutic.

In a preferred embodiment, the oral care composition is a monophase anhydrous composition. Typically the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the sensitive area. In liquid mouthwash form the composition may be packaged in a bottle, sachet or other convenient container.

The composition can be effective even when used in an individual's daily oral hygiene routine. For example, the composition may be brushed onto the teeth and/or be rinsed around the inside of the mouth of the individual. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day. When the oral care composition is a dual-phase composition, the two phases of the composition are mixed during application. The mixed phases are typically left on the teeth for from 3 minutes to 10 hours, more preferably from 3 minutes to 8 hours. The application may be carried out daily.

The following examples are provided to facilitate an understanding of the present invention.

### EXAMPLES

### Example 1

This example demonstrates the tooth whitening efficacy by using different particles. All ingredients are expressed by weight percent of the total formulation, and as level of active ingredient.

**Table 1**

| Ingredient | Samples | | | | | |
|---|---|---|---|---|---|---|
| | **1** | **2** | **3** | **4** | **5** | **6** |
| Calcium silicate^{a} | 40 | 40 | 40 | 40 | 40 | 40 |
| Titanium dioxide coated with calcium silicate^{b} | 60 | - | - | - | - | - |
| Titanium dioxide coated with alumina^{c} | - | 60 | - | - | - | - |
| Titanium dioxide coated with alumina^{d} | - | - | 60 | - | - | - |
| Titanium dioxide coated with alumina and hydrated silica^{e} | - | - | - | 60 | - | - |
| Titanium dioxide coated with hydrated alumina and silica^{f} | - | - | - | - | 60 | - |
| Titanium dioxide coated with silica^{g} | - | - | - | - | - | 60 |

| | | | | | | |
|---|---|---|---|---|---|---|
| a. Commercially available calcium silicate (CaSiO₃) from PQ Corporation (Sorbosil CA40) b. Commercially available titanium dioxide coated with calcium silicate from KOBO Products Inc c. Commercially available titanium dioxide coated with alumina under the trade name RC 402 from Sachtleben d. Commercially available titanium dioxide coated with alumina under the trade name MP-1133 from Tayca Corporation e. Commercially available titanium dioxide coated with alumina and hydrated silica under the trade name JR-800 from Tayca Corporation f. Commercially available titanium dioxide coated with hydrated alumina and silica under the trade name SIH-5 TiO2 R250 from KOBO Products Inc g. Commercially available titanium dioxide coated with silica under the trade name BTD-SiO₂-5 from KOBO Products Inc | | | | | | |

### Methods

To evaluate the tooth whitening efficacy of the samples, the following in vitro test was performed. Changes in whiteness just after the tooth brush is recorded as instant tooth whitening effect. The tooth color measurement was carried out using a Konica Minolta-2600D colorimeter. The WIO index is an index which has been optimized specifically for the evaluation of whiteness in teeth (as described in Journal of Dentistry, volume 36, Supplement 1, 2008, pages 2 to 7).

The test sample was mixed with water at a ratio of 2.5 g to 20 mL water to make a slurry. The bovine enamel blocks were treated with different slurries via brushing following the same protocol. The enamel blocks were brushed with the slurry under a tooth brushing machine equipped with toothbrushes. The load of the tooth brushing was 170g +/-5 g and the automatic brushing operated at a speed of 150 rpm. After brushing for 1 min, the tooth blocks were rinsed with 20 mL distilled water twice. The immediate tooth whitening was measured. The changes in WIO values (ΔWIO) from baseline were calculated and statistically analyzed.

### Results

The results of instant whitening after one brushing are summarized in table 2 (error represents 95% confidence interval for duplicate measurements).

**TABLE 2**

| Change in WIO | Samples | | | | | |
|---|---|---|---|---|---|---|
| | **1** | **2** | **3** | **4** | **5** | **6** |
| One Brushing | 4.44±2.48 | 7.37±2.07 | 4.44±2.30 | 10.48±2.41 | 9.88±2.59 | 7.48±2.07 |

Sample 1 is a comparative example comprising particles of titanium dioxide coated with calcium silicate. Samples 2 and 3, which comprise particles of titanium dioxide coated with alumina, showed comparable instant tooth whitening effect to Sample 1. Sample 6 comprising particles of titanium dioxide coated with silica also showed comparable performance to Sample 1. Unexpectedly, Samples 4 and 5 which comprise particles of titanium dioxide coated with alumina and hydrated silica and particles of titanium dioxide coated with hydrated alumina and silica respectively, showed much better instant tooth whitening effect than Sample 1. The WIO change of enamel blocks treated with Samples 4 and 5 showed a statistically significant increase (p<0.05) compared to those treated with Sample 1.

### Example 2

This example demonstrates the tooth whitening efficacy by using particles of different sizes. All ingredients are expressed by weight percent of the total formulation, and as level of active ingredient.

**TABLE 3**

| Ingredient | Samples | | |
|---|---|---|---|
| | **1** | **5** | **7** |
| Calcium silicate^{a} | 40 | 40 | 40 |
| Titanium dioxide coated with calcium silicate^{b} | 60 | - | - |
| Titanium dioxide coated with hydrated alumina and silica^{f} (270 nm) | - | 60 | - |
| Titanium dioxide coated with alumina and silica^{h} (500 nm) | - | - | 60 |

| | | | |
|---|---|---|---|
| h. Commercially available titanium dioxide coated with alumina and silica under the trade name R960 from DuPont | | | |

### Methods

To evaluate the tooth whitening efficacy of the samples, the following in vitro test was performed. The tooth color measurement was carried out using a Konica Minolta-2600D colorimeter.

The test sample was mixed with water at a ratio of 2.5 g to 20 mL water to make a slurry. The bovine enamel blocks were treated with different slurries via brushing following the same protocol. The enamel blocks were brushed with the slurry under a tooth brushing machine equipped with toothbrushes. The load of the tooth brushing was 170g +/-5 g and the automatic brushing operated at a speed of 150 rpm. After brushing for 1 min, the tooth blocks were rinsed with 20 mL distilled water twice. Then the enamel blocks were kept in simulated oral fluid (SOF) overnight (>12 hours) in a shaking water bath at 37°C to mimic oral environment, followed by 1 min water brushing to mimic the meals. These steps are considered as a whole treatment cycle within one day. After one brushing and one cycle of treatment, the changes in WIO values (ΔWIO) were measured by colorimeter and statistically analyzed.

Simulated oral fluid was made by combining the ingredients in Table 4:

**TABLE 4**

| Ingredient | Amount/g |
|---|---|
| NaCl | 16.07 |
| NaHCO₃ | 0.7 |
| KCl | 0.448 |
| K₂HPO₄*3H₂O | 3.27 |
| MgCl₂*6H₂O | 0.622 |
| 1M HCl | 40ml |
| CaCl₂ | 0.1998 |
| Na₂SO₄ | 0.1434 |
| Buffer | Adjust pH to 7.0 |
| Water | Balance to 2L |

### Results

The results after one brushing and one cycle of treatment are summarized in table 5 (error represents 95% confidence interval for duplicate measurements).

**TABLE 5**

| Change in WIO | Samples | | |
|---|---|---|---|
| | **1** | **5** | **7** |
| One Brushing | 5.82±0.66 | 7.84±0.62 | 10.26±0.76 |
| One Cycle | 15.04±1.21 | 20.96±2.79 | 17.95±1.94 |

It can be seen that after one brushing, both Samples 5 and 7 showed better instant tooth whitening effect than comparative Sample 1. The particles in Sample 5 have a particle size of around 270 nm while the particles in Sample 7 have a particle size of around 500 nm. Sample 5 showed obvious progressive tooth whitening effect compared to Sample 7.

After three cycles of treatments, Scanning Electron Microscopy (SEM) images of the enamel block surface were taken for all three samples, which demonstrated that a new layer was formed on the surface of the enamel block after 3 cycles of treatments. Analysis using Energy Dispersive X-ray Spectroscopy (EDX) indentified the elements of Ti, Ca and P within the new layer, indicating the deposition of TiO₂ particles on the tooth surface.

### Example 3

This example demonstrates the tooth whitening efficacy by using toothpaste comprising different particles. All ingredients are expressed by weight percent of the total formulation, and as level of active ingredient.

**TABLE 6**

| Ingredient | Samples | | |
|---|---|---|---|
| | **8** | **9** | **10** |
| Glycerin | 45.44 | 45.44 | 45.44 |
| PEG400 | 10.50 | 10.50 | 10.50 |
| Sodium monofluorophosphate | 1.11 | 1.11 | 1.11 |
| Sodium saccharin | 0.25 | 0.25 | 0.25 |
| Titanium dioxide coated with calcium silicate^{b} | 15.00 | - | - |
| Titanium dioxide coated with alumina^{d} | - | 15.00 | - |
| Titanium dioxide coated with hydrated alumina and silica^{f} | - | - | 15.00 |
| Calcium silicate^{a} | 10.00 | 10.00 | 10.00 |
| Silica abrasive | 6.00 | 6.00 | 6.00 |
| Sodium lauryl sulfate (70%) | 2.00 | 2.00 | 2.00 |
| Monosodium dihydrogen phosphate | 3.20 | 3.20 | 3.20 |
| Trisodium phosphate | 3.80 | 3.80 | 3.80 |
| Flavor | 1.20 | 1.20 | 1.20 |
| PEG3000 | 1.50 | 1.50 | 1.50 |

### Methods

To evaluate the tooth whitening efficacy of the samples, the following in vitro test was performed. The tooth color measurement was carried out using a Konica Minolta-2600D colorimeter.

The test sample was mixed with water at a ratio of 4 g toothpaste to 8 mL water to make a slurry. The bovine enamel blocks were treated with different slurries via brushing following the same protocol. The enamel blocks were brushed with the slurry under a tooth brushing machine equipped with toothbrushes. The load of the tooth brushing was 170 g +/-5 g and the automatic brushing operated at a speed of 150 rpm. After brushing for 10 seconds, the enamel blocks were incubated in the slurry for another 110 seconds, then rinsed by 40 mL distilled water. The enamel blocks were soaked in SOF in a shaking water bath at 37°C for 3 hours. These steps are considered as one brushing. After one brushing and twenty-eight times of brushing, the changes in WIO values (ΔWIO) were measured by colorimeter and statistically analyzed.

### Results

The results after one brushing and twenty-eight times of brushing are summarized in table 7 (error represents 95% confidence interval for duplicate measurements).

**TABLE 7**

| Change in WIO | Samples | | |
|---|---|---|---|
| | **8** | **9** | **10** |
| One brushing | 5.23±2.23 | 4.58±0.92 | 6.68±1.66 |
| 28 brushings | 5.12±1.96 | 6.35±2.51 | 10.30±1.76 |

It can be seen that Sample 9 showed comparable tooth whitening effect to Sample 8. But Sample 10 comprising particles of titanium dioxide coated with hydrated alumina and silica showed much better tooth whitening effect than Sample 8 after one brushing and twenty-eight times of brushing. The WIO change of enamel blocks treated with Sample 10 showed a statistically significant increase (p<0.05) compared to those treated with Sample 8.

## Claims

1. An oral care composition comprising:
a) calcium silicate;
b) composite particles; and
c) a physiologically acceptable carrier.
wherein the composite particle comprises a first component core comprising a metal compound having a refractive index in the range from 1.9 to 4.0, and a second component coating comprising a mixture of:
1) metal oxides and/or hydrated metal oxides; and
2) solid non-metal oxides and/or solid hydrated non-metal oxides; and wherein the metal oxide is alumina and the non-metal oxide is silica.

2. The oral care composition according to claim 1, wherein the metal compound is a metal oxide, preferably titanium dioxide, zinc oxide, zirconium dioxide or a mixture thereof.

3. The oral care composition according to claim 1 or 2, wherein the first component core of the composite particle is titanium dioxide.

4. The oral care composition according to claim 3, wherein the titanium dioxide has a crystal structure of anatase or rutile, preferably rutile.

5. The oral care composition according to any of the preceding claims, wherein the second component coating comprising a mixture of:
1) alumina and/or hydrated alumina; and
2) silica.

6. The oral care composition according to any of the preceding claims, wherein the second component coating comprising a mixture of:
1) alumina; and
2) silica and/or hydrated silica.

7. The oral care composition according to any of the preceding claims, wherein the second component coating comprises at least 60%, preferably 80 to 100% by weight of oxides and/or hydrated oxides.

8. The oral care composition according to any of the preceding claims, wherein the second component coating comprises 1) metal oxides and/or hydrated metal oxides and 2) solid non-metal oxides and/or solid hydrated non-metal oxides in a weight ratio ranging from 1:10 to 10:1, preferably from 1:5to 5:1.

9. The oral care composition according to any of the preceding claims, wherein the first component core and the second component coating is present in a weight ratio of at least 1:1, preferably at least 3:1, more preferably ranging from 5:1 to 30:1.

10. The oral care composition according to any of the preceding claims, wherein the composite particles have a particle size in the range from 50 nm to 1 micron, preferably from 150 nm to 350 nm.

11. The oral care composition according to any of the preceding claims, wherein the composite particle is present in an amount from 0.25 to 60% by weight of the composition, preferably from 1 to 30% by weight of the composition.

12. The oral care composition according to any of the preceding claims, wherein the calcium silicate is present in an amount from 0.1 to 50% by weight of the composition, preferably from 1 to 20% by weight of the composition.

13. The oral care composition according to any of the preceding claims, wherein the weight ratio of calcium silicate to the composite particle ranges from 1:3 to 2:1.

14. The oral care composition according to any of the preceding claims, wherein the composition further comprises a phosphate source selected from trisodium phosphate, monosodium dihydrogen phosphate, disodium hydrogen phosphate, sodium pyrophosphate, tetrasodium pyrophosphate, sodium hexametaphosphate, tripotassium phosphate, monopotassium dihydrogen phosphate, dipotassium hydrogen phosphate or a mixture thereof, preferably trisodium phosphate, monosodium dihydrogen phosphate or mixtures thereof.

15. The oral care composition according to any of the preceding claims, wherein the composition is a monophase anhydrous composition.

## Patentansprüche

1. Mundpflegezusammensetzung, umfassend:
a) Calciumsilicat;
b) Verbundpartikel; und
c) einen physiologisch verträglichen Träger,
wobei das Verbundpartikel umfasst einen Kern als ersten Bestandteil, umfassend eine Metallverbindung mit einem Brechungsindex in dem Bereich von 1,9 bis 4,0, und eine Beschichtung als zweiten Bestandteil, umfassend eine Mischung aus:
1) Metalloxiden und/oder hydratisierten Metalloxiden; und
2) festen Nichtmetalloxiden und/oder festen hydratisierten Nichtmetalloxiden, und
wobei das Metalloxid Aluminiumoxid und das Nichtmetalloxid Siliciumdioxid ist.

2. Mundpflegezusammensetzung nach Anspruch 1, wobei die Metallverbindung ein Metalloxid, vorzugsweise Titandioxid, Zinkoxid, Zirconiumdioxid oder eine Mischung davon, ist.

3. Mundpflegezusammensetzung nach Anspruch 1 oder 2, wobei der Kern als erster Bestandteil des Verbundpartikels Titandioxid ist.

4. Mundpflegezusammensetzung nach Anspruch 3, wobei das Titandioxid eine Kristallstruktur vom Anatas oder Rutil, vorzugsweise vom Rutil, aufweist.

5. Mundpflegezusammensetzung nach irgendeinem der vorhergehenden Ansprüche, wobei die Beschichtung als zweiter Bestandteil eine Mischung aus:
1) Aluminiumoxid und/oder hydratisiertem Aluminiumoxid und
2) Siliciumdioxid
umfasst.

6. Mundpflegezusammensetzung nach irgendeinem der vorhergehenden Ansprüche, wobei die Beschichtung als zweiter Bestandteil eine Mischung aus:
1) Aluminiumoxid und
2) Siliciumdioxid und/oder hydratisiertem Siliciumdioxid
umfasst.

7. Mundpflegezusammensetzung nach irgendeinem der vorhergehenden Ansprüche, wobei die Beschichtung als zweiter Bestandteil mindestens 60 Gewichts-%, vorzugsweise 80 bis 100 Gewichts-% Oxide und/oder hydratisierte Oxide umfasst.

8. Mundpflegezusammensetzung nach irgendeinem der vorhergehenden Ansprüche, wobei die Beschichtung als zweiter Bestandteil 1) Metalloxide und/oder hydratisierte Metalloxide und 2) feste Nichtmetalloxide und/oder feste hydratisierte Nichtmetalloxide in einem Gewichtsverhältnis, das von 1:10 bis 10:1, vorzugsweise von 1:5 bis 5:1, reicht, umfasst.

9. Mundpflegezusammensetzung nach irgendeinem der vorhergehenden Ansprüche, wobei der Kern als erster Bestandteil und die Beschichtung als zweiter Bestandteil in einem Gewichtsverhältnis von mindestens 1:1, vorzugsweise mindestens 3:1, vorliegen, bevorzugter von 5:1 bis 30:1 reichen.

10. Mundpflegezusammensetzung nach irgendeinem der vorhergehenden Ansprüche, wobei die Verbundpartikel eine Partikelgröße in dem Bereich von 50 nm bis 1 Mikron, vorzugsweise von 150 nm bis 350 nm, aufweisen.

11. Mundpflegezusammensetzung nach irgendeinem der vorhergehenden Ansprüche, wobei das Verbundpartikel in einer Menge von 0,25 bis 60 Gewichts-% der Zusammensetzung, vorzugsweise von 1 bis 30 Gewichts-% der Zusammensetzung, vorliegt.

12. Mundpflegezusammensetzung nach irgendeinem der vorhergehenden Ansprüche, wobei das Calciumsilikat in einer Menge von 0,1 bis 50 Gewichts-% der Zusammensetzung, vorzugsweise von 1 bis 20 Gewichts-% der Zusammensetzung, vorliegt.

13. Mundpflegezusammensetzung nach irgendeinem der vorhergehenden Ansprüche, wobei das Gewichtsverhältnis vom Calciumsilikat zu dem Verbundpartikel von 1:3 bis 2:1 reicht.

14. Mundpflegezusammensetzung nach irgendeinem der vorhergehenden Ansprüche, wobei die Zusammensetzung ferner eine Phosphatquelle umfasst, die aus Trinatriumphosphat, Mononatriumdihydrogenphosphat, Dinatriumhydrogenphosphat, Natriumpyrophosphat, Tetranatriumpyrophosphat, Natriumhexametaphosphat, Trikaliumphosphat, Monokaliumdihydrogenphosphat, Dikaliumhydrogenphosphat oder eine Mischung davon, vorzugsweise Trinatriumphosphat, Mononatriumdihydrogenphosphat oder Mischungen davon, ausgewählt ist.

15. Mundpflegezusammensetzung nach irgendeinem der vorhergehenden Ansprüche, wobei die Zusammensetzung eine einphasige wasserfreie Zusammensetzung darstellt.

## Revendications

1. Composition pour le soin oral comprenant :
a) du silicate de calcium ;
b) des particules de composite ; et
c) un support physiologiquement acceptable
dans laquelle la particule de composite comprend un noyau de premier constituant comprenant un composé de métal ayant un indice de réfraction dans l'intervalle de 1,9 à 4,0, et un revêtement de second constituant comprenant un mélange de :
1) oxydes de métaux et/ou oxydes de métaux hydratés ; et
2) oxydes de non-métaux solides et/ou oxydes de non-métaux solides hydratés ; et
dans laquelle l'oxyde de métal est l'alumine et l'oxyde de non-métal est la silice.

2. Composition pour le soin oral selon la revendication 1, dans laquelle le composé de métal est un oxyde de métal, de préférence le dioxyde de titane, l'oxyde de zinc, le dioxyde de zirconium ou un mélange de ceux-ci.

3. Composition pour le soin oral selon la revendication 1 ou 2, dans laquelle le noyau de premier constituant de la particule de composite est le dioxyde de titane.

4. Composition pour le soin oral selon la revendication 3, dans laquelle le dioxyde de titane présente une structure de cristal d'anatase ou de rutile, de préférence de rutile.

5. Composition pour le soin oral selon l'une quelconque des revendications précédentes, dans laquelle le revêtement de second constituant comprend un mélange de :
1) alumine et/ou alumine hydratée ; et
2) silice.

6. Composition pour le soin oral selon l'une quelconque des revendications précédentes, dans laquelle le revêtement de second constituant comprend un mélange de :
1) alumine ; et
2) silice et/ou silice hydratée.

7. Composition pour le soin oral selon l'une quelconque des revendications précédentes, dans laquelle le revêtement de second constituant comprend au moins 60 %, de préférence de 80 à 100 % en masse d'oxydes et/ou oxydes hydratés.

8. Composition pour le soin oral selon l'une quelconque des revendications précédentes, dans laquelle le revêtement de second constituant comprend 1) des oxydes de métaux et/ou oxydes de métaux hydratés et 2) des oxydes de non-métaux solides et/ou des oxydes de non-métaux solides hydratés dans un rapport en masse de 1:10 à 10:1, de préférence de 1:5 à 5:1.

9. Composition pour le soin oral selon l'une quelconque des revendications précédentes, dans laquelle le noyau de premier constituant et le revêtement de second constituant sont présents dans un rapport massique d'au moins 1:1, de préférence d'au moins 3:1, encore mieux de 5:1 à 30:1.

10. Composition pour le soin oral selon l'une quelconque des revendications précédentes, dans laquelle les particules de composite présentent une taille de particule dans l'intervalle de 50 nm à 1 micron, de préférence de 150 nm à 350 nm.

11. Composition pour le soin oral selon l'une quelconque des revendications précédentes, dans laquelle la particule de composite est présente dans une quantité de 0,25 à 60 % en masse de la composition, de préférence de 1 à 30 % en masse de la composition.

12. Composition pour le soin oral selon l'une quelconque des revendications précédentes, dans laquelle le silicate de calcium est présent dans une quantité de 0,1 à 50 % en masse de la composition, de préférence de 1 à 20 % en masse de la composition.

13. Composition pour le soin oral selon l'une quelconque des revendications précédentes, dans laquelle le rapport massique de silicate de calcium à la particule de composite est de 1:3 à 2:1.

14. Composition pour le soin oral selon l'une quelconque des revendications précédentes, dans laquelle la composition comprend de plus une source de phosphate choisie parmi le phosphate de trisodium, dihydrogénophosphate de monosodium, hydrogénophosphate de disodium, pyrophosphate de sodium, pyrophosphate de tétrasodium, hexamétaphosphate de sodium, phosphate de tripotassium, dihydrogénophosphate de monopotassium, hydrogénophosphate de dipotassium ou un mélange de ceux-ci, de préférence phosphate de trisodium, dihydrogénophosphate de monosodium ou mélanges de ceux-ci.

15. Composition pour le soin oral selon l'une quelconque des revendications précédentes, dans laquelle la composition est une composition anhydre de monophase.
